(19) Europäisches Patentamt
European Patent Office
Office européen des brevets

(11) EP 1 641 471 B1

(12) EUROPEAN PATENT SPECIFICATION

(45) Date of publication and mention of the grant of the patent:
**20.12.2006 Bulletin 2006/51**

(21) Application number: **03769399.1**

(22) Date of filing: **15.10.2003**

(51) Int Cl.:
*A61K 31/74* (2006.01) *A61F 2/02* (2006.01)
*C08F 290/06* (2006.01) *C08G 63/47* (2006.01)

(86) International application number:
**PCT/EP2003/011456**

(87) International publication number:
**WO 2005/002596 (13.01.2005 Gazette 2005/02)**

# (54) BIOCOMPATIBLE POLYMER NETWORKS

BIOKOMPATIBLE POLYMERNETZWERKE

RESEAUX POLYMERES BIOCOMPATIBLES

(84) Designated Contracting States:
**AT BE BG CH CY CZ DE DK EE ES FI FR GB GR HU IE IT LI LU MC NL PT RO SE SI SK TR**

(30) Priority: **03.07.2003 EP 03077105**

(43) Date of publication of application:
**05.04.2006 Bulletin 2006/14**

(73) Proprietor: **UNIVERSITEIT TWENTE**
**7522 NB Enschede (NL)**

(72) Inventors:
- **GRIJPMA, Dirk, Wybe**
  **NL-7556 CP Hengelo (NL)**
- **HOU, Qingpu**
  **Nottingham NG9 2GA (GB)**
- **FEIJEN, Jan**
  **NL-7552 GD Hengelo (NL)**

(74) Representative: **van Kooij, Adriaan et al**
**Arnold & Siedsma**
**Sweelickplein 1**
**2517 GK The Hague (NL)**

(56) References cited:
**US-A- 6 083 524** **US-A1- 2002 127 266**

- **MEIDONG LANG, CHIH-CHANG CHU: "Functionalized multiarm poly(-caprolactone)s: Synthesis, structure analysis, and network formation" JOURNAL OF APPLIED POLYMER SCIENCE, vol. 86, no. 9, 28 November 2002 (2002-11-28), pages 2296-2306, XP002275969**
- **ANTTI O. HELMINEN, HARRI KORHONEN, JUKKA V. SEPPÄLÄ: "Cross-Linked Poly(-caprolactone/D,L-lactide) Copolymers with Elastic Properties" MACROMOLECULAR CHEMISTRY AND PHYSICS, vol. 203, no. 18, December 2002 (2002-12), pages 2630-2639, XP002275970**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 641 471 B1

## Description

The present invention relates to functionalized prepolymers and to biocompatible polymer networks, especially biodegradable polymer networks, obtainable by polymerization of said functionalized prepolymers. The functionalized prepolymers and polymer networks are suitable for use as a medicament in for example the medical fields of tissue engineering and/or drug delivery. The invention further relates to methods for providing said functionalized prepolymers and said polymer networks.

Polymer networks play an important role in both human and veterinary medicine and especially in the medical fields of tissue engineering, repair and/or regeneration, and drug delivery.

The polymer networks can be used as temporary and/or biocompatible scaffolds in which cells and tissues can grow and proliferate. Such polymer networks are ideally in the form of creep-resistant elastomeric polymer networks.

The polymer network can serve as an in vivo scaffold providing for example an environment to the developing cells or tissues protecting the vulnerable cells or tissues against mechanical forces in the body, a basis for adherence of cells or tissues, or a mold for shaping the final form of the regenerated or engineered tissues.

These polymer networks can also be used in vitro, whereby first the cells or tissues are grown in a controlled environment like an incubator, and then transformed into the body. This approach is especially suited for cells or tissues which only grow under specific conditions, like $pO_2$, nutrient requirements, growth factors, or temperature. This approach also provides the person skilled in the art relative easy manipulation of the growing cells or tissues by for example genetic engineering in comparison with in vivo cell or tissue growth.

In addition, polymer networks, for example in the form of hydrogels, can be used for the delivery of a variety of therapeutic, prophylactic, and/or immunogenic compounds to the body or to specific target tissues. For this, the polymer network is loaded with one or more compounds, which usually are released either by diffusion out or by degradation of the polymer network or by combinations thereof.

One important aspect of the polymer networks is the biocompatibility of the network. Biocompatibility is determined by a number of factors amongst which the immunogenic properties of the polymer network, the mechanical properties of the polymer network, the degradation rate of the polymer network and very important, the biological toxicity of the network. Biological toxicity of a polymer network is predominantly determined by the toxicity of the compounds, the remnants, the reaction products, and/or the degradation products and the methods used for preparation.

In many cases polymer networks predominantly comprise polymerized, also designated as cross-linked, functionalized prepolymers, and the toxicity of the functionalized prepolymers, of their breakdown products, and of toxicity introduced into the network by for example additives during preparation play an important role in the final toxicity of the polymer network.

The term "prepolymers" as used herein comprises polymers and oligomers either linear, branched or star-shaped. A wide variety of prepolymers are available for providing polymer networks. A prepolymer can for example be a protein or protein complex, a polymer, a co-polymer, an oligonucleotide, a saccharide, an oligosaccharide, a polysaccharide, or combinations thereof.

Specific examples of prepolymers used in the art include poly(ethylene glycol) (PEG), poly(trimethylene carbonate) (polyTMC), poly(D,L-lactide) (PDDLA), poly(D-lactide) (PDLA), poly(L-lactide) (PLLA), poly(ε-caprolactone) (PCL), poly(ethylene glycol-lactide), poly(ethylene glycolcaprolactone), poly(ethylene glycol-glycolide), PEG-b-PLA, poly (D,L-3-methylglycolide)-PEG triblock copolymer, poly(ether-anhydride), PEG-PLLA or PCL multiblock copolymers, poly (ether ester)s, poly(ester-urethane) elastomers, poly(ester) elastomers, poly(hydroxybutyrate), poly(hydroxybutyrate-co-valerate), poly(phosphazenes), poly(glycerol sebacate), starch, or combinations thereof.

The term "functionalized prepolymer" as used herein comprises a derivatized prepolymer which derivatization usually consists of linking the prepolymer with one or more functional groups like (meth)acrylate, styryl, coumarin, phenylazide and fumarate groups. Such functionalized prepolymers allow for polymerization into polymer networks. In the art, a functionalized prepolymer is also designated as a macromer.

The degree and rate of polymerization, the properties of the functionalized prepolymers (macromers) used, and the specific reaction conditions all provide control of the characteristics of the resulting polymer network. For use in medicine desirable characteristics are for example creep resistance, structural integrity, degradation rate, or form, like a hydrogel or an elastomer.

Until now, polymer networks of functionalized prepolymers (macromers) still possess a certain undesirable degree of toxicity. As already stated above, this toxicity can be due to the prepolymer or its breakdown products, or to prepolymer, functionalized prepolymer and/or polymer network preparation methods. With respect to the preparation processes, the functional group used to functionalize the prepolymer, the specific method of functionalization, and/or the method used for the polymerization, for example the use of acrylates, can all contribute to the toxicity of the final product.

It is therefore an object of the present invention to provide a functionalized prepolymer (macromer) which can be used to provide polymer networks which polymer networks are less toxic, as compared with the polymer networks

according to the prior art, or non-toxic.

Therefore, according to the present invention, there is provided a functionalized prepolymer (macromer) obtainable by reaction of a prepolymer comprising at least one alcoholic, amine and/or sulfhydril group with an unsaturated mono-esterified dicarbonic acid.

Degradation of an unsaturated mono-esterified dicarbonic acid functionalized prepolymer (macromer) results in the release of non-toxic degradation products when the final product, the polymer network, is degraded. Unsaturated mono-esterified dicarbonic acids are therefore the functional group of choice over other (toxic) functional groups and their degradation products like (meth)acylate, styryl, coumarin, and phenylazide groups.

In addition, unsaturated mono-esterified dicarbonic acids allow for functionalization of the prepolymer under relatively mild conditions. These mild conditions are in general required since the prepolymers suitable to be used in the polymer network according to the invention are susceptible to degradation.

In the research that lead to the present invention, it was found that the use of other dicarbonic acids, like for example a carboxylic acid chloride, resulted in a deep brown color of the functionalized prepolymers (macromers) indicative of a degradation of the prepolymer during functionalization. This was probably due to the high reactivity of the carboxylic acid chloride. Similar results were obtained by using a dicarbonic acid without additional groups.

It was also surprisingly found by the inventors that prepolymers functionalized with an unsaturated mono-esterified dicarbonic acid showed a higher reactivity during formation of the polymer network in comparison with prepolymers functionalized with other dicarbonic acids.

In order to allow for a reaction, for example an ester reaction, between the unsaturated mono-esterified carbonic acid and the prepolymer, the prepolymer has to comprise a reactive group such as an alcohol, amine and/or sulfhydril group.

It is well within the knowledge of the person skilled in the art to determine a suitable reaction and reaction conditions to allow for a reaction between the unsaturated mono-esterified dicarbonic acid and the prepolymer resulting in a functionalized prepolymer (macromer). For example, if the prepolymer comprises an alcohol group, the reaction of choice will be an esterification reaction under the appropriate conditions like solvent, temperature, pH, etc.

A polymer network comprising the above-defined functionalized prepolymer (macromer) is obtainable by radical polymerization wherein the unsaturated bond in the unsaturated mono-esterified carbonic acid is used to link the separate derivatized macromers into a network.

Since it is preferable with respect to the desired characteristics of the polymer network to carefully control the polymerization, it is advantageous to use ultra-violet (UV) radical polymerization, optionally in combination with a photoinitiator, heat radical polymerization or redox initiation for the preparation of the polymer networks. By using these methods especially the rate of polymerization and the polymerization degree can be controlled.

In addition, since these polymerization methods require relatively mild reaction conditions, these methods of polymerization allow for the in vivo polymerization of the derivatized macromers which is advantageous when the resultant polymer network is to be prepared on or in the body.

According to one aspect of the present invention, the unsaturated mono-esterified dicarbonic acid is at a terminus of the prepolymer, thus providing an end-capped functionalized prepolymer (macromer). By using end-capped functionalized prepolymers (macromers), the resulting structure of the polymer networks and thus its characteristics like degradation and/or diffusion rates, and mechanical properties, can be further controlled since the way the functionalized prepolymers (macromers) are linked during the polymerization can be predicted in advance.

In one embodiment of the present invention, the unsaturated mono-esterified dicarbonic acid is mono-esterified fumaric acid. Breakdown products of fumaric acid are less or non-toxic and biocompatible and thus safe to use in polymer networks for tissue engineering, tissue regeneration, tissue repair, and/or drug delivery.

The unsaturated mono-esterified dicarbonic acid according to the invention is preferably obtained by esterification with a $C_1$-$C_5$ alcohol, more preferably an ethanol. These compounds exhibit an excellent solubility, are commercially available, and provide excellent reactivity of the functional group during polymerization.

Taken into account the above it is highly advantageous to functionalize the prepolymer into a functionalized prepolymer (macromer) according to the invention with fumaric acid mono-ethyl ester.

A large variety of prepolymers can be used according to the present invention, like proteins or protein complexes, polymers, co-polymers, oligonucleotides, sugars, oligosugars, polysugars, or combination thereof. Specific examples of prepolymers include, but not limited thereto, poly(ethylene glycol)(PEG), poly(trimethylene carbonate) (polyTMC), poly(D,L-lactide) (PDLLA), poly(D-lactide) (PDLA), poly(L-lactide) (PLLA), poly(ε-caprolactone) (PCL), poly(ethylene glycol-lactide), poly(ethylene glycolcaprolactone), poly(ethylene glycol-glycolide), PEG-b-PLA, poly(D,L-3-methylglycolide)-PEG triblock copolymer, poly(ether-anhydride), PEG-PLLA or PCL multiblock copolymers, poly(ether ester)s, poly (ester-urethane) elastomers, poly(ester) elastomers, poly(hydroxybutyrate), poly(hydroxybutyrate-co-valerate), poly (phosphazenes), poly(glycerol sebacate), or starch. The preferred prepolymers to be used according to the invention are poly(ethylene glycol) (PEG), poly(trimethylene carbonate) (TMC), poly(D,L-lactide), poly(ε-caprolactone), and/or poly(dioxanone).

According to the invention, the functionalized prepolymers (macromers) can be provided by a method comprising the reaction, for example an esterification, of at least one alcoholic, amine and/or sulfhydril group with an unsaturated mono-esterified dicarbonic acid. The specific reaction conditions used are common knowledge of the person skilled in the art and can be readily performed without undue experimentation.

For example, in case fumaric acid mono-ethyl ester is used, the reaction of choice will be an esterification reaction.

The polymer network according to the invention can be provided by a method comprising radical polymerization of the above-characterized functionalized prepolymers (macromers). For reasons already provided, the radical polymerization is preferably ultra-violet (UV) radical polymerization, redox radical polymerization, and/or heat radical polymerization. The functionalized prepolymers (macromers) can be in the form of a melt, solution and/or suspension.

According to one embodiment, a method for providing a polymer network according to the invention comprises

- dissolution of the functionalized prepolymer (macromer) in a suitable solvent or providing a melt of the functionalized prepolymer (macromer);
- ultra-violet (UV) radiation, redox radical polymerization, and/or heat treatment of the functionalized prepolymer (macromer).

Dissolution of the functionalized prepolymer (macromer) is preferably carried out in a solvent which evaporates at low temperatures for easy removal of the solvent after processing. Examples of such solvents are chloroform, dichloromethane, THF, and acetone.

After dissolution or melting the functionalized prepolymer (macromer), optionally one or more (photo)initiators can be used for starting the radical polymerization cascade. These (photo)initiators can be added to the dissolved functionalized prepolymer (macromer) or the melt before, during, or after dissolution or melting. Examples of suitable (photo)initiators according to the present invention are 2,2-dimethoxy-2-phenylacetophenone (DMPA), peroxide, and AIBN.

If a solution of the functionalized prepolymer (macromer) is used, the solvent can optionally be evaporated before polymerization.

The resultant solution, film (either obtained from evaporation of the solvent or cooling of the melt), and/or other structures, is exposed to ultra-violet (UV) light and/or heat in order to polymerize the functionalized prepolymers (macromers). This exposure is continued until a polymer network with the desired characteristics is obtained.

The polymer network according to the invention is especially suited to be used as a medicament. Such medicament can be used for tissue engineering, tissue regeneration, and/or cell or drug delivery.

One example of the use of the polymer network according to the invention for tissue engineering is to design a polymer network defining pre-determined cavities. Such cavities can be obtained by adding to the mixture of the functionalized prepolymer (macromer) and the (photo)initiator, leachable particles like salts. After polymerization these particles are removed by leaching resulting in a polymer network with cavities wherein the size and distribution of the cavities is determined by the method of mixing and polymerization and the size of the leachable particles.

After obtaining such polymer network, cells or tissue preparations can be seeded in or on the polymer network under suitable conditions and allowed to grow and/or to develop either in vivo or in vitro

In addition, it is also possible to use advantageously the functionalized prepolymers (macromers) as a medicament as such. The functionalized prepolymer (macromer), either in solution, as a liquid, as a suspension, as a gel, or as a solid, can be placed on or inside the body. Subsequently polymerization of the functionalized prepolymer can be initiated forming a polymer network according to the invention.

The polymer network obtained can be used as, for example, a scaffold for tissue generation and/or repair or for the delivery of compounds to the body by mixing into the functionalized prepolymer composition a therapeutic, immunogenic or prophylactic compounds thus providing for example a sustained release matrix.

The invention will further be illustrated in the following examples which are not intended to limit the embodiments of the present invention, but are provided for illustration purposes only. In the examples reference is made to the following figures.

## FIGURES

**Figure 1.** $^1$H-NMR spectrum of a TMC/DLLA functionalized prepolymer (macromer) in $CDCl_3$.

**Figure 2.** Thermal properties of TMC/DLLA triols, functionalized prepolymers (macromers) and the resultant networks as a function of TMC content in the oligomers.

**Figure 3.** Phase transition behavior of linear 2000PEG50/PMTC50 diol.

**Figure 4.** DSC traces of 2000PEG50/PMTC50 diol, functionalized prepolymer (macromer) and the resultant networks (A) and 1000OPEG50/PMTC50 triol, functionalized prepolymer (macromer) and the resultant network (B).
**Figure 5.** Swelling behavior of the resultant PEG/PTMC networks as a function of temperature.
**Figure 6.** Synthesis route for three-armed ethyl fumarated end-capped trimethylene carbonate prepolymer.
**Figure 7.** Creep-recovery curve of linear high molecular weight PTMC and TMC networks ($M_n$ 13,9 * $10^3$) formed by UV photo crosslinking (after extraction in ethanol).
**Figure 8.** Scaffold prepared from the functionalized prepolymer (macromer)/salt mixture by photo crosslinking (salt particle size: 250-425 μm; salt concentration: 75 wt%);. A: $M_n$ 4.5*$10^3$; B: $M_n$ 9.4*$10^3$.

## EXAMPLES

The values indicated are expressed in their corresponding SI-units unless stated otherwise. Specifically, number average molecular weights ($M_n$) are expressed in $10^3$ g/mol units, unless stated otherwise.

### Example 1. Synthesis of biocompatible polymer networks by UV photo crosslinking

### Introduction

Below, the synthesis is described of biocompatible, and especially biodegradable, networks formed by photo cross-linking of ethyl fumarate end-capped functionalized prepolymers (macromers). The functionalized prepolymers (macromers) were obtained through the reaction of hydroxyl-terminated prepolymers and fumaric acid monoethyl ester under mild conditions. Prepolymers (oligomers) from D,L-lactide, 1,3-trimethylene carbonate (TMC), ε-caprolactone (CL) and ethylene glycol (co)monomers were used for the synthesis of these functionalized prepolymers (macromers) and polymer networks. The absorbable networks are designed to eventually convert to non-toxic degradation products.

### Experimental

Materials

D,L-lactide and 1,3-trimethylene carbonate (TMC) (1,3-dioxan-2-one) were obtained from Purac, the Netherlands and Boehringer Ingelheim, Germany, respectively. D,L-lactide was purified by recrystallization under dry $N_2$ from sodium dried toluene. ε-caprolactone (CL, Acros Organics, Belgium) was purified by drying over $CaH_2$ and distilled under reduced argon atmosphere. Stannous octoate ($SnOct_2$) was used as received from Sigma, USA. Glycerol (spectrophotometric grade), fumaric acid monoethyl ester and 4-(dimethylamino)pyridine (DMAP) were purchased from Aldrich. N,N-dicyclohexylcarbodiimide (DCC) was purchased from Fluka. Dichloromethane (Biosolve, the Netherlands) was dried over $CaH_2$ and distilled. 2,2-dimethoxy-2-phenylacetophenone ((DMPA, Aldrich) was used as a photo initiator. Petroleum ether (b.p. 40-60 ˚C) was purchased from Merck (Germany). Poly(ethylene glycol) ($M_n$=2.0*$10^3$ and, 4.0 *$10^3$ g/mol), poly(ε-caprolactone) ($M_n$=1.3*$10^3$, and 2.0*$10^3$ g/mol) oligomers were obtained from Aldrich.

Synthesis of linear and branched ethyl fumarate end-capped functionalized Prepolymers (macromers)

The synthesis of linear and 3-armed ethyl fumarate end-capped functionalized prepolymers (macromers) were prepared by esterification of the corresponding oligomer diols or triols with fumaric acid monoethyl ester in the presence of DMAP and DCC at room temperature.
Poly(ethylene glycol) and poly(ε-caprolactone) diols are commercially available. Three-armed poly(trimethylene carbonate-co-D,L-lactide) and poly(trimethylene carbonate-co-ε-caprolactone) triols are synthesized by ring-opening polymerization of TMC and DLLA or CL mixtures.
A typical reaction was carried out as follows. In an argon atmosphere, (co)monomer(s), glycerol, and 2*$10^{-4}$ mol of stannous octoate per mol of (co)monomer were added into a three-necked flask with a magnetic stirrer. The molecular weights of the polymer triols were varied by adjusting the (co)monomer/ glycerol ratio. The reaction was carried out at 130 ˚C under stirring for 40 hr. Then the reaction mixture was cooled to room temperature and dissolved in dichloromethane. The product was purified by precipitation in an excess of petroleum ether to remove the unreacted monomer and dried in a vacuum oven for 2 days at room temperature.
Ethyl fumarate end-capped macromers were prepared as follows. For example, three-armed (trimethylene carbonate-coD,L-lactide) oligomer triol (0.001 mol) was charged in a 100 ml three-necked flask equipped with a magnetic stirrer. The oligomeric triol was dried by heating at 110 ˚C under vacuum for 6 hr. After cooling to room temperature, 60 ml of dried dichloromethane was added. The contents of the reaction flask were kept under a dry argon atmosphere at room temperature while stirring. Then 0.0036 mol of fumaric acid monoethyl ester was added to the triol solution. The mixture was stirred for another 30 minutes before a chloroform solution containing DCC (0.0036 mol) and DMAP (0.0001

mol) was added drop-wise during vigorous stirring. The reaction was continued at room temperature for 48 hr. During the reaction, dicyclohexylurea (DCU) was precipitated as a white solid. The precipitate was removed by filtration, and the filtrate was precipitated in an excess of petroleum ether. The product was recovered by filtration and dried in a vacuum oven for 40 hr at room temperature to a constant weight.

Photo-crosslinking of the functionalized Prepolymers (macromers)

Functionalized prepolymers (macromers) and photoinitiator were mixed in chloroform, cast, and after solvent evaporation the resulting transparent films were exposed to UV light (15 W, 360nm UV-tube light; Philips, The Netherlands). The distance between the UV lamp and the sample was 10 cm. Photo-crosslinking time was controlled for 3 hr. After photo-crosslinking, the gel content of the resultant networks was measured. The gel content was defined as the percentage of insoluble part against the total weight of the irradiated structure before extraction. Extraction was performed in chloroform at room temperature for 24 hr.

$$\text{Gel content} = w_1/w_2 \times 100\%$$

Wherein $w_1$ is the weight of insoluble part after extraction and $w_2$ the total weight of the inadiated structure before extraction.

Characterization

The synthesized prepolymers (oligomers) and functionalized prepolymers (macromers) were characterized with respect to the monomer conversion and chemical composition by nuclear magnetic resonance (NMR) spectroscopy. 300 MHz $^1$H-NMR (Varian Inova 300 MHz) spectra were recorded using prepolymer (oligomer) or functionalized prepolymer (macromer) solutions in $CDCl_3$ (Sigma, USA).

Thermal properties of the prepolymers (oligomers) and resultant polymer networks were evaluated by differential scanning calorimetry (DSC). Samples (5-15 mg) placed in stainless pans were analysed with a Perkin Elmer DSC-7 at a heating rate of 10 ˚C/min. All samples were heated to 100 ˚C. The samples were then quenched rapidly (300 ˚C/min) until -80 ˚C and after 5 min a second scan was recorded. Unless indicated otherwise, the data presented were taken collected during the second heating scan. The glass transition temperature was taken as the midpoint of the heat capacity change. Indium and gallium were used as standards for temperature calibration.

Molecular weights, molecular weight distributions, and intrinsic viscosities of the triol polymers and macromers were determined by gel permeation chromatography (GPC) using a Waters Model 510 pump, a HP-Ti-Series 1050 autosampler, a Waters Model 410 Differential Refractomer, and a Viscotek H502 Viscometer Detector with $10^5$-$10^4$-$10^3$-510 Å Waters Ultra-Styragel columns placed in series. Chloroform was used as eluent at a flow rate of 1.5 ml $min^{-1}$. Narrow polystyrene standards were used for calibration. Sample concentrations of approximately 0.5% wt/vol and injection volume of 30 μl were used. All determinations were performed at 25 ˚C.

**Results**

Synthesis of ethyl fumarate end-capped prepolymers

To synthesize the biocompatible, and especially biodegradable, polymer networks, ethyl fumarate end-capped prepolymers were first prepared from linear PEG and PCL diols, and star-shaped TMC-co-DLLA and TMC-co-CL triols. The diols are commercially available and used directly, while the star-shaped triols were synthesized by ring-opening polymerization of the (co)monomer at 130 ˚C using glycerol as an initiator and stannous octoate as a catalyst. The syntheses and characteristics of the TMC/DLLA and TMC/CL triols are illustrated in Tables 1 and 2.

**Table 1.** Synthesis and characteristics of TMC/DLLA triols.

| Code | TMC:DLLA monomer (mol%) | TMC conversion (%) | DLLA conversion (%) | TMC:DLLA polymer (mol%) | $M_n$ | PDI | $T_g$ (˚C) | Appearance at RT |
|---|---|---|---|---|---|---|---|---|
| 1 | 0:100 | - | 96.9 | 0:100 | 4.0 | 1.22 | 30.0 | brittle, solid |

(continued)

| Code | TMC:DLLA monomer (mol%) | TMC conversion (%) | DLLA conversion (%) | TMC:DLLA polymer (mol%) | $M_n$ | PDI | $T_g$ (˚C) | Appearance at RT |
|---|---|---|---|---|---|---|---|---|
| 2 | 20:80 | 96.8 | 96.9 | 14:86 | 4.0 | 1.27 | 16.4 | glassy, transparent |
| 3 | 40:60 | 96.5 | 99.6 | 31:69 | 3.8 | 1.38 | 4.9 | viscous, transparent |
| 4 | 60:40 | 99.6 | 94.2 | 53:97 | 3.1 | 1.49 | -9.6 | viscous, transparent |
| 5 | 80:20 | 97.9 | 96.3 | 76:24 | 3.3 | 1.63 | -21.2 | viscous, transparent |

**Table 2.** Synthesis and characteristics of TMC/CL triols.

| Code | TMC:CL monomer (mol%) | TMC conversion (%) | CL conversion (%) | TMC:CL polymer (mol%) | $M_n$ | PDI | $T_g$ (˚C) | $T_c$ (˚C) | $T_m$ (˚C) | Appearance at RT |
|---|---|---|---|---|---|---|---|---|---|---|
| 1 | 20:80 | 100 | 100 | 20:80 | 4.0 | 1.77 | -63.0 | -35.1 | 16.0, 30.3 | waxy |
| 2 | 40:60 | 100 | 100 | 39:61 | 3.8 | 1.54 | -60.0 | - | - | viscous |

The conversions of monomer TMC and DLLA or CL and the composition of the resultant copolymer were determined from the $^1$H-NMR spectra of the crude products. The α-methylene protons in the oligomer triols were shifted to 4.18-4.26 ppm from 4.44 ppm for the original α-methylene protons from trimethylene carbonate. The α-methylene protons in the oligomer triols were shifted to 1.39-1.63 ppm from 1.65-1.67 ppm for the original α-methylene protons from D,L-lactide.

The monomer conversion for the polymerization was determined using the integral intensities of the corresponding monomeric and oligomeric peaks. Similarly the conversions of TMC and CL during the co-polymerization were also determined, as the α-methylene resonances of monomeric (m, 2H, δ=2.52-2.67ppm) and polymeric (m, 2H, δ=2.23-2.40ppm) CL were separated. Under the reaction conditions applied, the conversion of the monomer was almost complete.

The molecular weight of the triol can be controlled by (co)monomers and glycerol feed ratio, as confirmed by GPC results. The designed molecular weight of all these triols was 3512 g/mol. The composition of the triols (with different molar content of TMC unit) can be tuned by changing the (co)monomer ratio. In this manner TMC/DLLA and TMC/CL oligomer triols with controlled molecular weights and compositions were obtained.

The glass transition temperature of the triols decreases with increasing the content of TMC content in the oligomer triols. This is in good agreement with linear high molecular weight poly(TMC/DLLA) copolymers. The oligomer triols varied from viscous liquid to waxy or brittle solid at room temperature depending on their composition.

The ethyl fumarate functionalization of the linear PEG and PCL diols and star-shaped TMC/DLLA and TMC/CL triols were carried out through the reaction with fumaric acid monoethyl ester at room temperature. The reaction was under mild conditions using DMAP as a catalyst and DCC as a coupling agent. The functionalization was confirmed by $^1$H-NMR spectra. A typical $^1$H-NMR spectrum of TMC/DLLA functionalized prepolymer (macromer) is shown in Figure 1. The thermal properties of the functionalized prepolymers (macromers) were similar to their precursor diols or triols.

<u>Photo crosslinking of the ethyl fumarate end-capped prepolymers</u>

The photo crosslinking of the ethyl fumarate end-capped prepolymers was performed using a UV tube with 360 nm wavelength. The functionalized prepolymer (macromer) films were prepared from their chloroform solution. First the photo initiator concentration and UV irradiation time on the gel content of the resultant polymer networks were studied. The highest gel content was obtained when 1wt% photo initiator and 3hrs UV irradiation time were applied. Therefore all the experiments were carried out under these conditions. Linear PCL and PEG and star shaped TMC/DLLA and TMC/CL prepolymers terminated with ethyl fumarate groups were used for the synthesis of biodegradable networks. Gel contents of the resultant biodegradable networks ranging from 67% to 96% were obtained for these linear and star-shaped functionalized prepolymers (macromers). The characteristics of the obtained polymer networks are summarized in tables 3-5.

**Table 3.** Synthesis and characteristics of TMC/DLLA macromers and networks.

| Code | TMC:DLLA polymer (mol%) | $M_n$ | PDI | Yield (%) | $T_g$ of macromer (˚C) | Appearance at RT | Gel content (%) | $T_g$ of networks (˚C) |
|---|---|---|---|---|---|---|---|---|
| 1 | 0:100 | 4.7 | 1.17 | 74.0 | 34.3 | brittle | 81.4 | 38.6 |
| 2 | 20:80 | 4.4 | 1.44 | 72.0 | 17.3 | glassy | 77.8 | 23.0 |
| 3 | 40:60 | 4.2 | 1.39 | 74.0 | 7.0 | sticky | 75.8 | 14.0 |
| 4 | 60:40 | 2.9 | 1.49 | 68.2 | -8.7 | viscous | 67.3 | 12.0 |
| 5 | 80:20 | 3.7 | 1.16 | 69.6 | -13.8 | viscous | 72.6 | 1.4 |

**Table 4.** Synthesis and characteristics of TMC/CL macromers and networks.

| Code | $M_n$ | PDI | Yield (%) | Appearance at RT | $T_9$ of macromer (˚c) | $T_e$ (˚C) | $T_m$ (˚C) | Gel conten t (%) | $T_g$ of networks (˚c) | $T_c$ of networks (˚C) | $T_m$ of networks (˚c) |
|---|---|---|---|---|---|---|---|---|---|---|---|
| 1 | 4.8 | 1.54 | 71.2 | waxy | -59.1 | -19.5 | 9.5, 29.2 | 86.1 | -49.7 | -13.8 | 28.6 |
| 2 | 4.0 | 1.72 | 69.3 | viscous | -53.7 | - | - | 79.8 | -44.5 | - | - |

**Table 5.** Synthesis and characteristics of PCL and PEG macromers and the resultant networks.

| | Yield of macromer (%) | $T_g$ of macromer (˚C) | $T_m$ of macromer (˚C) | Gel content (%) |
|---|---|---|---|---|
| PCL2000 | 71.0 | -57.4 | 50.3 | 68.0 |
| PCL1250 | 66.0 | -57.5 | 42.0, 48.8 | 76.7 |
| PEG4000 | 73.0 | -37.4 | 53.6 | 96.2 |
| PEG2000 | 68.0 | -35.8 | 40.5 | 88.6 |

Figure 2 shows the thermal properties of TMC/DLLA triols, functionalized prepolymers (macromers) and the resultant networks as a function of TMC content in the oligomers. It can be seen that the glass transition temperatures of the resultant polymer networks increased compared to their corresponding functionalized prepolymers (macromers). In the case of semi-crystalline functionalized prepolymers (macromers), the melting temperatures of the finals networks decreased compared to their macromers.

As the glass transition temperature and melting temperature of the functionalized prepolymers (macromers) can be readily controlled by variation of their composition, the possibility is provided of varying the thermal properties of the final polymer networks.

**Conclusion**

Synthesis of networks was developed by UV photo crosslinking of ethyl fumarate end-capped PEG, PCL, poly (trimethylene carbonate-co-D,L-lactide), and poly(trimethylene carbonate-co-ε-caprolactone) macromers.

The linear or star-shaped functionalized prepolymers (macromers) were prepared by ethyl fumarate functionalization of hydroxyl-terminated prepolymers under mild conditions. The biocompatible, and especially biodegradable, networks are designed to release only non-toxic degradation products.

**Example 2. Biocompatible elastomeric PEG/PTMC hydrogels prepared by UV photo crosslinking**

**Introduction**

This example describes the synthesis of biocompatible, and especially biodegradable, elastomeric hydrogels based on 1,3-trimethylene carbonate and poly(ethylene glycol) by UV photo crosslinking. To obtain such hydrogels ethyl fumarate groups were linked to the chain-ends of oligo(ethylene glycol-co-trimethylene carbonate)diols or triols.

**Experimental**

Materials

1,3-trimethylene carbonate (TMC) (1,3-dioxan-2-one) was obtained from Boehringer Ingelheim, Germany. Stannous octoate ($SnOct_2$) was used as received from Sigma, USA. Fumaric acid monoethyl ester and 4-(dimethylamino) pyridine (DMAP) were purchased from Aldrich.

N,N-dicyclohexylcarbodiimide (DCC) was purchased from Fluka. Dichloromethane (Biosolve, the Netherlands) was dried over $CaH_2$ and distilled. 2,2-dimethoxy-2-phenylacetophenone ((DMPA, Aldrich) was used as a non toxic photoinitiator. Petroleum ether (b.p. 40-60 ˚C) was purchased from Merck (Germany). Poly(ethylene glycol) (PEG) (Mn=$2*10^3$ g/mol) was obtained from Fluka. Branched poly(ethylene glycol) (3-arm, MW=$10*10^3$ g/mol) was purchased from Shearwater corporation.

Synthesis of linear and branched ethyl fumarate end-capped prepolymers

The synthesis of linear and 3-armed ethyl fumarate end-capped prepolymers were prepared by ring-opening polymerization of 1,3-trimethylene carbonate using linear or branched PEG as an initiator and stannous octoate as a catalyst followed by esterification of the corresponding oligomer diol or triol with fumaric acid monoethyl ester in the presence of DMAP and DCC at room temperature.

A typical reaction was carried out as follows. In an argon atmosphere, linear or branched poly(ethylene glycol), 1,3-trimethylene carbonate, and $2*10^{-4}$ mol of stannous octoate per mol of monomer were added into a three-necked

flask. The molecular weights of the polymer diols or triols were varied by adjusting the monomer/ poly(ethylene glycol) ratio. The reaction was carried out at 130 ˚C under stirring for 40 hr. Then the reaction mixture was cooled to room temperature and dissolved in dichloromethane. The product was purified by precipitation in an excess of petroleum ether to remove the unreacted monomer. The products were then dried in a vacuum oven for 2 days at room temperature.

Ethyl fumarate end-capped prepolymers were prepared as follows. The functionalization of branched 10000PEG50PTMC50 triols is taken as an example. Three-armed PEG/PTMC oligomer triol (0.0001 mol) was charged in a 50 ml three-necked flask equipped with a magnetic stirrer. The oligomeric triol was dried by heating at 130 ˚C under vacuum for 6 hr. After cooling to room temperature, 30 ml of dried dichloromethane was added. The contents of the reaction flask were kept under a dry argon atmosphere at room temperature while stirring. Then 0.00036 mol of fumaric acid monoethyl ester was added to the triol solution. The mixture was stirred for another 30 minutes before a chloroform solution containing DCC (0.00036 mol) and DMAP (0.00001 mol) was added drop-wise during vigorous stirring. The reaction was continued at room temperature for 48 hr. During the reaction, dicyclohexylurea (DCU) was precipitated as a white solid. The precipitate was removed by filtration, and the filtrate was precipitated in an excess of petroleum ether. The product was recovered by filtration and dried in a vacuum oven for 40 hr at room temperature to a constant weight.

Photo-crosslinking of the functionalized Prepolymers (macromers)

Functionalized prepolymer (macromer) and photoinitiator were mixed in chloroform, cast, and after solvent evaporation, the resulting transparent films were exposed to UV light (15 W, 360nm UV-tube light; Philips, The Netherlands). The distance between the UV lamp and the sample was 10 cm. Photo-crosslinking time was controlled for 3 hr. After photo-crosslinking, the gel content of the resultant networks was measured.

The gel content is defined as the percentage of insoluble part against the total weight of the crosslinked structure before extraction. Extraction was performed in chloroform at room temperature for 24 hr.

$$\text{Gel content} = w_1/w_2 \times 100\%$$

Wherein $w_1$ is the weight of insoluble part after extraction and $w_2$ the total weight of the inadiated structure before extraction.

Characterization

The synthesized prepolymers and functionalized prepolymers (macromers) were characterized with respect to the monomer conversion and chemical composition by nuclear magnetic resonance (NMR) spectroscopy. 300 MHz $^1$H-NMR (Varian Inova 300 MHz) spectra were recorded using oligomer or macromer solutions in $CDCl_3$ (Sigma, USA).

Thermal properties of the oligomers, functionalized prepolymers (macromers), and resultant polymer networks were evaluated by differential scanning calorimetry (DSC). Samples of 5-15 mg were placed in stainless pans and were analysed with a Perkin Elmer DSC-7 at a heating rate of 10 ˚C/min. All samples were heated to 100 ˚C. The samples were then quenched rapidly (300 ˚C/min) until -80 ˚C and after 5 min a second scan was recorded. Unless mentioned otherwise, the data presented were taken collected during the second heating scan. The glass transition temperature was taken as the midpoint of the heat capacity change. Indium and gallium were used as standards for temperature calibration.

Tensile testing

The tensile strength and elongation at break of the polymer networks were obtained at room temperature using a Zwick tensile tester equipped with a 500 N load cell at a crosshead speed of 50 mm/min.

**Results**

Synthesis of ethyl fumarate end-capped prepolymers

To synthesize the amphiphilic biodegradable polymeric networks, ethyl fumarate end-capped prepolymers were prepared by ring-opening polymerization 1,3-trimethylene carbonate at 130˚C in the presence of linear or branched PEG, and subsequent functionalization of the resultant diol or triol.

The conversions of monomer TMC were determined from the $^1$H-NMR spectra of the crude products. The $\alpha$-methylene protons in the oligomer diol and triol were shifted to 4.18-4.26 ppm from 4.44 ppm for the original ?-methylene protons from trimethylene carbonate. The monomer conversion for the polymerization was determined using the integral

intensities of the corresponding monomeric and oligomeric peaks.

Under the reaction conditions applied, the conversion of the monomer was almost complete (99.8 and 97.2% initiated with linear PEG 2000 and 3-armed PEG 10000, respectively).

Table 6 shows the synthesis and characteristics of linear and branched PEG/PTMC oligomeric diol/ triol. It was observed that the chemical composition of the diol or triol was in good agreement with the feed ratio. This correlates with the high TMC conversion during the ring-opening polymerization. The oligomeric diol and triol were waxy solid at room temperature. The linear 2000PEG50PTMC50 can be dissolved in water, while branched 10000 PEG50PTMC50 is water insoluble. The phase transition behavior of linear 2000PEG50PTMC50 diol is illustrated in Figure 3. When the concentration of the diol aqueous solution reached to 30 wt%, it formed a gel in the temperature range of 4-30 ˚C. Upon increasing temperature to 34 ˚C the gel started to flow. Further increase in temperature led to the precipitation of the polymer.

**Table 6.** Synthesis and characteristics of linear and branched PEG/PTMC oligomeric diol/triol

| PEG/PTMC | Linear 2000PEG50PTMC50 | Branched 10000PEG50PTMC50 |
|---|---|---|
| Molecular weight of PEG | 2000 | 10000 |
| Feed ratio, PEG:TMC (wt) | 50:50 | 50:50 |
| Conversion of TMC (%) | 99.8 | 97.2 |
| Composition* PEG:PTMC (wt) | 50:50 | 52:48 |
| Water solubility | soluble | insoluble |
| $T_g$ (˚C) | -44.1 | -44.7 |
| $T_c$ (˚C) | -17.9 | -17.4 |
| $T_n$ (˚C) | 38.6 | 44.0 |
| *determined by $^1$H-NMR analysis | | |

The ethyl fumarate functionalization of the linear PEG/PTMC diol and star-shaped PEG/PTMC triol was carried out through the reaction with fumaric acid monoethyl ester at room temperature. The reaction was under mild conditions using DMAP as a catalyst and DCC as a coupling agent.

Yields of more than 80% of the products were obtained. $^1$H-NMR analysis of the resultant functionalized prepolymers (macromers) showed resonances at ??? 6.80-6.84ppm (A, A'), 2H; 4.20-4.24ppm (B), 2H; 1.25-1.29ppm (C), 3H, respectively, confirming the esterification reaction between the hydroxyl group of the diol or triol and the carboxyl group of the fumaric acid monoethyl ester. A,A', B, and C are defined in the molecule as follows.

$$-O-CO-\underset{A}{CH}=\underset{A'}{CH}-COO\underset{B}{CH_2}\underset{C}{CH_3}$$

The thermal properties of the functionalized prepolymers (macromers) were similar to their precursor linear diol or branched triol, as shown in Table 7 and Figure 4. The linear 2000PEG50PTMC50 macromer was soluble in water. The macromer solution displays similar thermosensitivity behavior as the linear 2000PEG50PTMC50 diol. With increasing the concentration of the macromer, the cloud points of the linear macromer increased.

**Table 7.** Characteristics of linear and branched PEG/PTMC functionalized prepolymers (macromers) and the resultant networks.

| | Linear 2000PEG50PTMC50 | | branched 10000PEG50PTMC50 | |
|---|---|---|---|---|
| | macromer | network | macromer | network |
| Water solubility | soluble | insoluble | insoluble | insoluble |
| $T_g$ (˚C) | -43.3 | -38.8 | -43.4 | -42.5 |

(continued)

| | Linear 2000PEG50PTMC50 | | branched 10000PEG50PTMC50 | |
|---|---|---|---|---|
| | macromer | network | macromer | network |
| $T_c$ (˚C) | -16.0 | -7.2 | -20.4 | -17.8 |
| $T_m$ (˚C) | 37.9 | 40.5 | 46.8 | 44.9 |
| Gel content (%) | - | 94.5 | - | 90.7 |

Photo crosslinking of the ethyl fumarate end-capped macromers

The photo crosslinking of the ethyl fumarate end-capped prepolymers was performed using a UV tube with 360 nm wavelength. The functionalized prepolymer (macromer) films were prepared from their chloroform solutions. First the photo initiator concentration and UV irradiation time on the gel content of the resultant polymer networks were studied. The highest gel content was obtained when the concentration of photo initiator was 1wt% and the UV irradiation time was 3hrs. All the experiments were carried out under these conditions.

Gel contents of the resultant biodegradable networks were 94.5 and 90.7% for the linear and star-shaped macromers.

The glass transition temperatures of the resultant polymer networks increased compared to their corresponding macromers. In the resultant networks, the crystallization temperature and melting temperature were slightly shifted (see Figure 4). The crystallinity of the networks largely decreased. It can be attributed to the inhibition crystallization on the cross-linking.

The swelling behavior of the resultant PEG/PTMC networks as a function of temperature is shown in Figure 5. It was observed that the swelling capacity of the 2000PEG50PTMC50 networks was much lower than that of the 10000PEG50PTMC50 networks, even though the weight percentage of the hydrophilic component was comparable. The longer PEG chain in the latter networks may result in a higher water uptake.

In both of the two networks the swelling capacity decreased with increasing temperature. This is because the hydrophobic interactions dominate when increasing temperature. For a fixed structure of the copolymer networks, the swelling capacity can thus be accurately controlled by temperature.

The mechanical properties of the resultant networks in both dry and wet states are shown in Table 8. It can be seen that in both cases the branched 10000PEG50PTMC50 was more flexible compared to the linear 2000PEG50PTMC networks, possessing lower modulus and much higher elongation at break. In the hydrated state, the mechanical properties of the crosslinked networks significantly decreased.

**Table 8.** Mechanical properties of the resultant PEG/PTMC networks.

| network | state | E-modulus (MPa) | Tensile strength (MPa) | Elongation at break (%) |
|---|---|---|---|---|
| linear 2000PEG50PTMC50 | dry | 94.3 | 3.0 | 3.1 |
| linear 2000PEG50PTMC50 | wet | 3.97 | 0.99 | 32.6 |
| branched 10000PEG50PTMC50 | dry | 66.1 | 14.0 | 47.4 |
| branched 10000PEG50PTMC50 | wet | 3.16 | 1.47 | 37.7 |

**Conclusions**

Biocompatible, and especially biodegradable, elastomeric hydrogels have been synthesized by UV photo crosslinking of ethyl fumarate end-capped prepolymers. The functionalized prepolymers (macromers) were prepared by ring-opening polymerization of trimethylene carbonate using linear or branched poly(ethylene glycol) as an initiator and stannous octoate as a catalyst, followed by esterification with fumaric acid monoethyl ester in the presence of N, N-dicyclohexylcarbodiimide (DCC) and 4-dimethylamino pyridine (DMAP) at room temperature. The resultant polymer networks displayed thermosensitve properties and can be used in biomedical fields.

**Example 3. Creep-resistant biodegradable poly(trimethylene carbonate) elastomer networks by UV photo-crosslinking.**

**Introduction**

This example describes the synthesis of biocompatible, and especially biodegradable, elastomeric networks formed by photo cross-linking of ethyl fumarated end-capped prepolymers containing trimethylene carbonate.
The functionalized prepolymers (macromers) were obtained through the reaction of hydroxyl-terminated trimethylene carbonate prepolymers and fumaric acid monoethyl ester under mild conditions.

**Experimental**

Materials

1,3-trimethylene carbonate (TMC) (1,3-dioxan-2-one) was obtained from Boehringer Ingelheim, Germany. Stannous octoate $(SnOct)_2$ was used as received from Sigma, USA. Glycerol (spectrophotometric grade), fumaric acid monoethyl ester and 4-(dimethylamino) pyridine (DMAP) were purchased from Aldrich. N,N-dicyclohexylcarbodiimide (DCC) was purchased from Fluka. Dichloromethane (Biosolve, the Netherlands) was dried over $CaH_2$ and distilled. 2,2-dimethoxy-2-phenylacetophenone (DMPA) (Aldrich) was used as a nontoxic photo-initiator. Petroleum ether (b.p. 40-60 ˚C) was purchased from Merck (Germany). Linear high molecular weight PTMC ($M_n$=300, 000 g/mol, $M_w$=530,000 g/mol, $T_g$= -13.9 ˚C) was used as a control.

Synthesis of 3-arm ethyl fumarate end-capped Prepolymers

The synthesis of the 3-arm ethyl fumarate end-capped trimethylene carbonate prepolymers includes two steps: preparation of 3-arm hydroxyl terminated trimethylene carbonate oligomers by ring-opening polymerization and functionalization of the oligomers by the reaction with fumaric acid monoethylene ester, as depicted in figure 6.
Three-armed polymer triols were synthesized by ring-opening polymerization of TMC. A typical reaction was carried out as follows. In an argon atmosphere, 1,3-trimethylene carbonate, glycerol, and $2 * 10^{-4}$ mol of stannous octoate per mol of monomer were added into a three-necked flask equipped with a magnetic stirrer. The molecular weights of the polymer triols were varied by adjusting the monomer/ glycerol ratio.
The reaction was carried out at 130 ˚C under stirring for 40 hr. Then the reaction mixture was cooled to room temperature and dissolved in dichloromethane. The product was purified by precipitation in an excess of petroleum ether to remove the unreacted monomer and dried in a vacuum oven for 2 days at room temperature.
Three-armed ethyl fumarate end-capped functionalized prepolymers (macromers) were prepared through the reaction of 3-armed polymer triols with fumaric acid monoethyl ester in the presence of DCC and DMAP at room temperature. The procedure for the functionalization of the triols is as follows: Three-armed trimethylene carbonate oligomer triol (Mn 6000, 0.001 mol) was charged in a 100 ml three-necked flask equipped with a magnetic stirrer. The oligomeric triol was dried by heating at 120 ˚C under vacuum for 6 hr. After cooling to room temperature, 60 ml of dried dichloromethane was added. The contents of the reaction flask were kept under a dry argon atmosphere at room temperature while stirring. Then 0.0036 mol of fumaric acid monoethyl ester was added to the triol solution. The mixture was stirred for another 30 minutes before DCC (0.0036 mol) and DMAP (0.0001 mol) were added during vigorous stirring. The reaction was continued at room temperature for 48 hr. During the reaction, dicyclohexylurea (DCU) was precipitated as a white solid. The precipitate was removed by filtration, and the filtrate was precipitated in an excess of petroleum ether. The product was recovered by filtration and dried in a vacuum oven for 40 hr at room temperature to a constant weight.

Photo-crosslinking of the functionalized prepolymers (macromers)

Functionalized prepolymer (macromer) and photoinitiator were mixed in chloroform, cant, and after solvent evaporation the resulting transparent films were exposed to UV light (15 W, 360nm UV-tube light; Philips, Holland). The distance between the UV lamp and the sample was 10 cm. Photo-crosslinking time was controlled for 3 hr. After photo-crosslinking, the gel content of the resultant networks was measured. The gel content was defined as the percentage of insoluble part against the total weight of the inadiated structure before extraction. Extraction was performed in chloroform at room temperature for 24 hr.

$$\text{Gel content} = w_1/w_2 \times 100\%$$

Wherein $w_1$ is the weight of the insoluble part after extraction and $w_2$ the total weight of the inadiated structure before extraction.

The swelling properties of the networks were also measured in chloroform and ethanol, after immersing the samples in the solvent for 24 hr.

Porous structures prepared from the functionalized prepolymers (macromers) by photo-crosslinking in the presence of salt particles

Porous structures were prepared from the functionalized prepolymers (macromers) by photo-crosslinking in the presence of leachable salt particles (250-425 μm). Functionalized prepolymer (macromer), salt, and photo initiator were premixed in chloroform. After the solvent evaporation the mixture was subject to UV irradiation with wavelength of 360 nm for 3 hr. The crosslinked network composites were placed in gently stirred demineralized water for a period of 4-5 days to leach out the salt. After drying, porous structures were obtained.

Characterization

The synthesized prepolymers (oligomers) and functionalized prepolymers (macromers) were characterized with respect to the monomer conversion and chemical composition by nuclear magnetic resonance (NMR) spectroscopy. 300 MHz $^1$H-NMR (Varian Inova 300 MHz) spectra were recorded using prepolymer or functionalized prepolymer (macromer) solutions in $CDCl_3$ (Sigma, USA) with tetramethyl silane (TMS) as internal reference.

Thermal properties of the prepolymers (oligomers), functionalized prepolymers (macromers), and resultant polymer networks were evaluated by differential scanning calorimetry (DSC). Samples of 5-15 mg were placed in stainless pans and analyzed with a Perkin Elmer DSC-7 at a heating rate of 10 ˚C/min. All samples were heated to 100 ˚C. The samples were then quenched rapidly (300 ˚C/min) until -80 ˚C and after 5 min a second scan was recorded. Unless mentioned otherwise, the data presented were taken collected during the second heating scan.

The glass transition temperature was taken as the midpoint of the heat capacity change. Indium and gallium were used as standards for temperature calibration.

Molecular weights, molecular weight distributions, and intrinsic viscosities of the triol polymers and functionalized prepolymers (macromers) were determined by gel permeation chromatography (GPC) using a Waters Model 510 pump, a HP-Ti-Series 1050 autosampler, a Waters Model 410 Differential Refractomer, and a Viscotek H502 Viscometer Detector with $10^5$-$10^4$-$10^3$-510 Å Waters Ultra-Styragel columns placed in series. Chloroform was used as eluent at a flow rate of 1.5 ml min$^{-1}$. Narrow polystyrene standards were used for calibration. Sample concentrations of approximately 0.5% wt/vol and injection volume of 30 μl were used. All determinations were performed at 25 ˚C.

Tensile testing

The tensile strength and elongation at break of the polymer networks were obtained at room temperature using a Zwick tensile tester equipped with a 10 N load cell at a crosshead speed of 50 mm/min. The constant creep rate was calculated from the strain-time curve when the samples were loaded to a standard stress (10% yield stress).

Cyclic tensile testing

Cyclic tensile testing was carried out at room temperature. The specimens (50 mm in length, 5 mm in width and 0.1 mm in thickness) were drawn up to 50% strain at a rate of 50 mm/min, and then the load was removed. The following cycles started and ended at the same points as the first cycle. After 20 cycles, the specimens were allowed to recover for 2hr, before 21$^{st}$ cycle was performed. The permanent set was determined at the beginning of the 21$^{st}$ cycle. Linear high molecular weight PTMC was used as a control.

Creep-recovery test

A static type creep test was performed under application of a load corresponding to 40% yield stress of the materials, recording the strain as a function of time at room temperature. After 34 hr the load was removed and again the strain was measured as a function of time. Linear high molecular weight PTMC was also used as a control.

Scanning electron microscopy (SEM)

A Hitachi S800 scanning electron microscope was used to examine the morphology of the porous scaffolds. Cross-sections of the scaffolds were coated with gold using a sputter-coater (Turbo Sputter Coater E6700, UK).

Results

Four TMC oligomer triols with different molecular weights were synthesized using glycerol as a ring-opening reagent and are shown in Table 9.

**Table 9.** Synthesis and characteristics of TMC triols

| Code | $M_n$ (theoretical) | TMC[a] conversion (%) | $M_n$[b] | PDI[b] | $T_g$[c] (°C) | Appearance at RT |
|---|---|---|---|---|---|---|
| 1 | 4.5 | 97.6 | 4.3 | 1.61 | -28.6 | viscous, transparent |
| 2 | 6.0 | 98.2 | 5.6 | 1.53 | -25.1 | gummy, transparent |
| 3 | 9.0 | 98.8 | 8.7 | 1.41 | -23.9 | gummy, transparent |
| 4 | 15.0 | 98.7 | 13.0 | 1.28 | -20.7 | sticky, transparent |
| a) Calculated from $^1$H-NMR<br>b) Determined by GPC analysis with calibrated polystyrene standards<br>c) Measured by DSC | | | | | | |

The monomer conversion was determined by $^1$H-NMR analysis of the crude polymerization products. The α-methylene protons in the oligomer triols were shifted to 4.18-4.26 ppm from 4.44 ppm for the original α-methylene protons from trimethylene carbonate. The monomer conversion for the polymerization was determined using the integral intensities of these two peaks. Under the reaction conditions applied, the conversions of the monomer were over 97%.

The molecular weight (ranging from $4.3*10^3$ to $13.0*10^3$) of the triol can be controlled by variation of the monomer/ glycerol ratio, as confirmed by GPC results. It was noted that the polydispersity index slightly became smaller with increasing the molecular weight of the triols. In this manner transparent TMC oligomer triols ranging from viscous liquids to sticky semi-solids were obtained.

The glass transition temperature of the triols was increased from -28.6 to -20.7 °C by increasing the molecular weight of the triols.

In the following step, the functionalized prepolymer (macromer) synthesis was carried out by reaction of TMC oligomer triols with fumaric acid monoethyl ester in the presence of DCC and DMAP at room temperature. The functionalization was carried out in dichloromethane under mild conditions and yields of more than 80% of the final products were obtained (Table 10).

**Table 10.** Molecular weights and appearance of the ethyl fumarate end-capped TMC prepolymers.

| Code | $M_n$ | PDI | Appearance at RT | Yield (%) |
|---|---|---|---|---|
| 1 | 4.5 | 1.69 | viscous | 70.1 |
| 2 | 5.8 | 1.46 | viscous | 75.8 |
| 3 | 9.4 | 1.19 | waxy | 75.5 |
| 4 | 13.9 | 1.19 | solid | 87.0 |

In table 11 is displayed the attribution of $^1$H-NMR peaks for TMC oligomer triols and macromers. $^1$H-NMR spectra confirmed the esterification reaction between carboxyl group in fumaric acid monoethyl ester and hydroxyl group in TMC oligomer triols.

**Table 11.** $^1$H-NMR assignments

| Oligomer | | Chemical shifts |
|---|---|---|
| TMC triol | 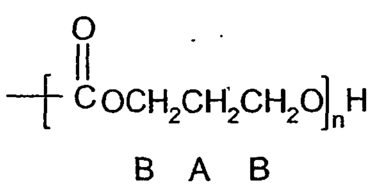 | A: 1.7-2.2ppm, 2H;<br>B: 3.9-4.2ppm, 4H |

(continued)

| Oligomer | | Chemical shifts |
|---|---|---|
| TMC macromer | 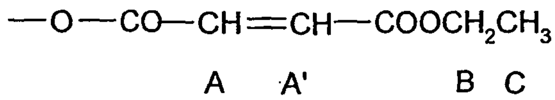 | A, A': 6.80-6.84ppm, 2H; B: 4.20-4.24ppm, 2H; C: 1.25-1.29ppm, 3H |

In IR-spectra a median absorption band at 1648 $cm^{-1}$, which was related to C=C stretching of the ethyl fumarate group, was observed after the functionalization. With increasing molecular weight, the resultant TMC macromers appear from viscous liquids to waxy or solids at room temperature. The functionalized prepolymer (macromer) films cast from chloroform were transparent.

The ethyl fumarate end-capped prepolymers were crosslinked by UV light irradiation at 360nm wavelength.

The formation of the crosslinked networks was confirmed by IR spectra and gel content of the networks. After crosslinking of the functionalized prepolymers (macromers), the absorption from the double bond in the macromers around 1648 $cm^{-1}$ disappeared.

The effects of photo initiator concentration and UV irradiation time on the gel content of the resultant networks were studied. It was found that higher gel content could be obtained when 1 wt% photo initiator and 3hr irradiation time were applied. Therefore all other experiments were carried out under these conditions.

The gel content of the resultant networks and swelling ratio of the networks in chloroform and ethanol are shown in Table 12.

**Table 12.** Gel content and swelling properties of the polymer networks (360 nm, 3hr, DMPA as photo initiator, 1 wt%)

| Code | $M_n$ | Gel content (%) | Swelling ratio in chloroform (%) | Swelling ratio in ethanol (%) |
|---|---|---|---|---|
| 1 | 4.5 | 79.4 | 500 | 7 |
| 2 | 5.8 | 77.8 | 570 | 8 |
| 3 | 9.4 | 74.6 | 1150 | 13 |
| 4 | 13.9 | 73.9 | 1200 | 16 |

It can be seen that with increasing of the molecular weight of the original functionalized prepolymers (macromers), the gel content of the resultant networks slightly decreased under the same conditions. It was also found that the swelling ratio of the resultant networks was increased with increasing the molecular weight of the functionalized prepolymers (macromers) in both chloroform and ethanol. This results from the higher crosslinking density in the networks with shorter chain length of the functionalized prepolymers (macromers).

The swelling ratio of the resultant networks in chloroform (500-1200%) is approximately 70 times higher than that in ethanol (7-16%).

The thermal properties of TMC functionalized prepolymers (macromers) and the resultant networks are shown in Table 13.

**Table 13.** Thermal properties of the macromers and the resultant networks.

| Macromer | $M_n$ | $T_g$ of macromer (˚C) | $T_g^1$ of network (˚C) | $T_g^2$ of network (˚C) | $T_g^3$ of network (˚C) |
|---|---|---|---|---|---|
| 1 | 4.5 | -23.2 | -15.8 | -14.5 | -13.1 |
| 2 | 5.8 | -22.6 | -17.0 | -14.3 | -13.3 |
| 3 | 9.4 | -19.9 | -18.1 | -17.8 | -13.6 |
| 4 | 13.9 | -20.0 | -14.7 | -13.0 | -13.3 |
| 1) before extraction<br>2) after extraction in chloroform<br>3) after extraction in ethanol | | | | | |

All TMC functionalized prepolymers (macromers) were amorphous. It can be seen that with increasing the molecular weight of the macromers the glass transition temperature slightly increased. The glass transition temperature of the resultant networks increased about 2-7 ˚C compared to their corresponding functionalized prepolymers (macromers). After extraction with chloroform or ethanol the $T_g$s were further increased by about 1-4 ˚C.

The tensile strength and elongation at break of the networks increased with the molecular weight of the macromers. When the molecular weight of the macromer was increased up to 13.9*$10^3$ g/mol, the tensile strength and elongation at break of the crosslinked networks was significantly increased up to 14.3 MPa and 750%, respectively (Table 14). Except the elongation at break of the networks prepared from the macromer with a molecular weight of 13.9*$10^3$, the mechanical properties were improved after extraction of the resultant networks in ethanol, as shown in Table 14.

**Table 14.** Mechanical properties of TMC networks formed by UV photo crosslinking.

| $M_n$ macromer | Network[1] | E-modulus (MPa) | Tensile strength $R_m$ (MPa) | Elongation at break $\varepsilon_{max}$ (%) |
|---|---|---|---|---|
| 4.5 | BE | 1.2 | 1.0 | 100 |
| 4.5 | AE | 2.1 | 1.5 | 130 |
| 5.8 | BE | 1.1 | 1.6 | 230 |
| 5.8 | AE | 1.8 | 2.9 | 210 |
| 9.4 | BE | 1.0 | 1.8 | 370 |
| 9.4 | AE | 2.0 | 3.4 | 380 |
| 13.9 | BE | 1.8 | 14.3 | 750 |
| 13.9 | AE | 2.3 | 14.9 | 750 |

[1)] BE: before extraction; AE: after extraction

Cyclic tensile experiments showed that all the PTMC networks prepared by UV photo crosslinking before and after extraction in ethanol were very elastic, possessing zero permanent set.

Under the same experimental conditions, 5.0% permanent set was observed after 20 consecutive deformation cycles in the linear high molecular weight PTMC.

In linear high molecular weight PTMC the flow of chain segment is more likely to occur than in the crosslinked networks especially under external stress. In other words, photo crosslinking restricts the flow of the chain segments under loading conditions and favors the creep-resistance. Also, strain-induced crystallization was detected in linear high molecular weight PTMC by DSC, while in the PTMC networks created by UV irradiation, there was no strain-induced crystallization observed.

The result of a static type creep-recovery test performed under loads corresponding to 40% yield stress as a function of time is shown in Figure 7. It can be seen that the PTMC networks show much lower creep rates than that of linear high molecular weight PTMC. During the 34 hrs loading period, the creep deformation of the crosslinked networks increased with time only in the first 2hrs, then remained constant at 30% strain for the rest period. While in the case of the linear high molecular weight PTMC, the creep deformation continues with time up to about 400 % strain.

Most importantly, after removing the load, the amount of recovery of the PTMC networks is significantly higher than the linear counterpart. After about 2-3 hr full recovery to their original length was observed in the crosslinked networks, while there was still about 230% of strain remaining even after 24 hrs for the linear high molecular weight PTMC.

Therefore the creep resistance of the photo crosslinked PTMC networks has been much improved compared to the linear high molecular weight PTMC. As their glass transition temperatures were comparable, the marked difference in creep-recovery behavior may result from different structures. Due to the presence of physical entanglement of polymer chains, the glass transition temperature of linear high molecular weight PTMC can be as high as that of cross-linked networks. However, under continuous loading conditions, the chain segments in the former were susceptible to disentanglement.

The flow of chain segments in chemically cross-linked networks was much more restricted under the same conditions. As a result the creep-resistance of the cross-linked networks was remarkably improved.

Porous scaffolds can be readily prepared from these ethyl fumarate-end capped prepolymers by UV photo crosslinking in the presence of salt particles, followed by leaching of the salt particles.

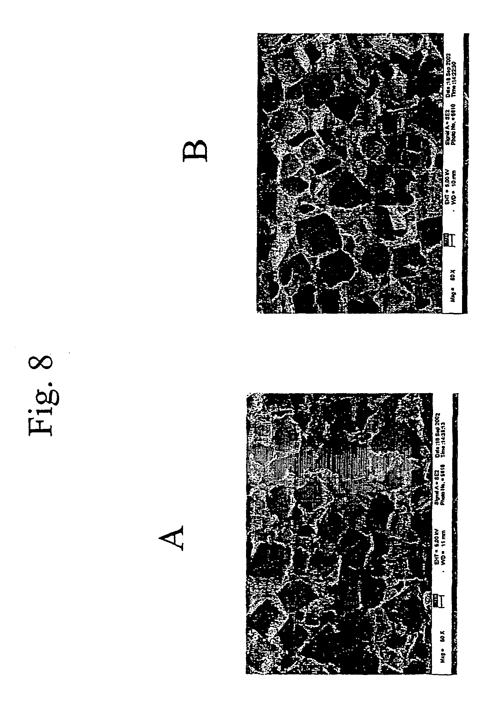

Figure 8 shows the porous scaffolds prepared in this manner. The initial salt content was 75 wt% and salt particle size was in the range of 250-425 μm. It was found that the introduction of the salt particles into the mixture did

not influence the UV photo crosslinking process of the functionalized prepolymers (macromers) significantly. The resultant pore morphology reflected the shape and size of the salt particles used. The porosity was close to the theoretical value. Therefore, the pore size and porosity of the resultant porous structures can be well controlled by variation of the salt particle size range and the salt weight fraction in the mixture, respectively.

The mechanical properties of porous scaffolds depend on the materials used as well as their porosity and pore structure. As the mechanical properties of the TMC networks prepared by photo crosslinking are largely dependent on the molecular weight of the functionalized prepolymers (macromers), the mechanical properties of the scaffolds can be tuned by varying the molecular weight of the functionalized prepolymers (macromers), the porosity of the scaffold or pore morphology. Therefore this technique facilitates the optimization of porous structures, which can be used in tissue regeneration or in medicine fields.

## Conclusion

Biodegradable rubbery networks were developed by UV photo crosslinking of 3-arm ethyl fumarate end-capped trimethylene carbonate prepolymers (oligomers). The functionalized prepolymers (macromers) were prepared by ring-opening polymerization of trimethylene carbonate in the presence of glycerol as an initiator and stannous octoate as a catalyst, followed by ethyl fumarate functionalization under mild conditions. The resultant networks were highly elastic and creep-resistant.

Porous structures were also readily prepared from the functionalized prepolymers (macromers) by photo-crosslinking in the presence of leachable salt particles followed by salt leaching. The resultant elastomer networks are to be used in soft-tissue engineering as well as in other biomedical fields.

## Claims

1. Functionalized prepolymer (macromer) obtainable by reaction of a prepolymer comprising at least one alcohol, amine, and/or sulfhydril group, with an unsaturated mono-esterified dicarbonic acid.

2. Functionalized prepolymer (macromer) according to claim 1, wherein the prepolymer is end-capped with the unsaturated mono-esterified dicarbonic acid.

3. Functionalized prepolymer (macromer) according to claim 1 or claim 2, wherein the unsaturated mono-esterified dicarbonic acid is mono-esterified fumaric acid.

4. Functionalized prepolymer (macromer) according to any of the claims 1-3, wherein the unsaturated mono-esterified dicarbonic acid is esterified with a $C_1$-$C_5$ alkyl alcohol, preferably an ethyl alcohol.

5. Functionalized prepolymer (macromer) according to any of the claims 1-4, wherein the unsaturated mono-esterified dicarbonic acid is fumaric acid monoethyl ester.

6. Functionalized prepolymer (macromer) according to any of the claims 1-5, wherein the prepolymer is chosen from the group consisting of poly(ethylene glycol) (PEG), poly(trimethylene carbonate) (polyTMC), poly(D,L-lactide) (PDLLA), poly(L-lactide) (PLLA), poly(D-lactide) (PDLA), poly(ε-caprolactone) (PCL), poly(dioxanone), and combinations thereof.

7. Polymer network obtainable by radical polymerization of a functionalized prepolymer (macromer) according to any of the claims 1-6.

8. Polymer network according to claim 7, wherein the radical polymerization is ultra-violet (UV) radical polymerization, redox radical polymerization, and/or heat radical polymerization.

9. Method for providing a functionalized prepolymer (macromer), comprising reacting of a prepolymer comprising at least one alcohol, amine, and/or sulfhydril group with an unsaturated mono-esterified dicarbonic acid

10. Method according to claim 9, wherein the alcohol, amine, and/or sulfhydril group is present at the terminus of the prepolymer.

11. Method according to claim 9 or claim 10, wherein the unsaturated mono-esterified dicarbonic acid is mono-esterified

fumaric acid.

12. Method according to any of the claims 9-11, wherein the unsaturated mono-esterified dicarbonic acid is esterified with a $C_1$-$C_5$ alkyl alcohol, preferably an ethyl alcohol.

13. Method according to any of the claims 9-12, wherein the unsaturated mono-esterified dicarbonic acid is fumaric acid monoethyl ester.

14. Method according to any of the claims 9-13, wherein the prepolymer is chosen from the group consisting of poly (ethylene glycol) (PEG), poly(trimethylene carbonate) (polyTMC), poly(D,L-lactide) (PDLLA), poly(L-lactide) (PLLA), poly(D-lactide) (PDLA), poly(ε-caprolactone) (PCL), poly(dioxanone), and combinations thereof.

15. Method for providing a polymer network comprising radical polymerization of a functionalized prepolymer (macromer) as defined in any of the claims 1-6.

16. Method according to claim 15, wherein radical polymerization is ultra-violet (UV) radical polymerization, redox radical polymerization, and/or heat radical polymerization.

17. Method according to claim 15 or claim 16 comprising:

- dissolution of the functionalized prepolymer (macromer) in a suitable solvent or providing a melt of the functionalized prepolymer (macromer);
- ultra-violet (UV) radiation, redox, and/or heat treatment of the functionalized prepolymer (macromer).

18. Use of a polymer network as defined in claim 7 or claim 8 as a medicament.

19. Use of a functionalized prepolymer (macromer) as defined in any of the claims 1-6 as a medicament.

## Patentansprüche

1. Funktionalisiertes Vorpolymer (Makromer), erhältlich durch Reaktion eines Vorpolymers, das mindestens eine Alkohol-, Amin- und/oder Sulfhydrylgruppe umfasst, mit einer ungesättigten einfachveresterten Dicarbonsäure.

2. Funktionalisiertes Vorpolymer (Makromer) gemäß Anspruch 1, wobei das Vorpolymer mit der ungesättigten einfachveresterten Dicarbonsäure am Ende abgeschlossen ist.

3. Funktionalisiertes Vorpolymer (Makromer) gemäß Anspruch 1 oder Anspruch 2, wobei die ungesättigte einfachveresterte Dicarbonsäure einfachveresterte Fumarsäure ist.

4. Funktionalisiertes Vorpolymer (Makromer) gemäß einem der Ansprüche 1 bis 3, wobei die ungesättigte einfachveresterte Dicarbonsäure mit einem $C_1$-$C_5$-Alkylalkohol, bevorzugt einem Ethylalkohol, verestert ist.

5. Funktionalisiertes Vorpolymer (Makromer) gemäß einem der Ansprüche 1 bis 4, wobei die ungesättigte einfachveresterte Dicarbonsäure Fumarsäuremonoethylester ist.

6. Funktionalisiertes Vorpolymer (Makromer) gemäß einem der Ansprüche 1 bis 5, wobei das Vorpolymer aus der Gruppe aus Poly(ethylenglycol) (PEG), Poly(trimethylencarbonat) (PolyTMC), Poly(D,L-lactid) (PDLLA), Poly(L-lactid) (PLLA), Poly(D-lactid) (PDLA), Poly(ε-caprolacton) (PCL), Poly(dioxanon) und deren Kombinationen ausgewählt ist.

7. Polymemetzwerk, erhältlich durch radikalische Polymerisation eines funktionalisierten Vorpolymers (Makromer) gemäß einem der Ansprüche 1 bis 6.

8. Polymernetzwerk gemäß Anspruch 7, wobei die radikalische Polymerisation ultraviolette (UV) radikalische Polymerisation, Redox-radikalische Polymerisation und/oder Wärme-radikalische Polymerisation ist.

9. Verfahren zum Bereitstellen eines funktionalisierten Vorpolymers (Makromer), das die Umsetzung eines Vorpolymers, das mindestens eine Alkohol-, Amin- und/oder Sulfhydryl-Gruppe umfasst, mit einer ungesättigten einfachveresterten Dicarbonsäure umfasst.

**10.** Verfahren gemäß Anspruch 9, wobei die Alkohol-, Amin- und/oder SulfhydrylGruppe an dem Ende des Vorpolymers vorhanden ist.

**11.** Verfahren gemäß Anspruch 9 oder 10, wobei die ungesättigte einfachveresterte Dicarbonsäure einfachveresterte Fumarsäure ist.

**12.** Verfahren gemäß einem der Ansprüche 9 bis 11, wobei die ungesättigte einfachveresterte Dicarbonsäure mit einem $C_1$-$C_5$-Alkylalkohol, bevorzugt einem Ethylalkohol, verestert ist.

**13.** Verfahren gemäß einem der Ansprüche 9 bis 12, wobei die ungesättigte einfachveresterte Dicarbonsäure Fumarsäuremonoethylester ist.

**14.** Verfahren gemäß einem der Ansprüche 9 bis 13, wobei das Vorpolymer aus der Gruppe aus Poly(ethylenglycol) (PEG), Poly(trimethylencarbonat) (PolyTMC), Poly(D,L-lactid) (PDLLA), Poly(L-lactid) (PLLA), Poly(D-lactid) (PDLA), Poly(ε-caprolacton) (PCL), Poly(dioxanon) und deren Kombinationen ausgewählt ist.

**15.** Verfahren zum Bereitstellen eines Polymernetzwerks, das die radikalische Polymerisation eines funktionalisierten Vorpolymers (Makromer) gemäß einem der Ansprüche 1 bis 6 umfasst.

**16.** Verfahren gemäß Anspruch 15, wobei die radikalische Polymerisation ultraviolette (UV) radikalische Polymerisation, Redox-radikalische Polymerisation und/oder Wärme-radikalische Polymerisation ist.

**17.** Verfahren gemäß Anspruch 15 oder Anspruch 16, das umfasst:

- Auflösung des funktionalisierten Vorpolymers (Makromer) in einem geeigneten Lösungsmittel oder Bereitstellen einer Schmelze des funktionalisierten Vorpolymers (Makromer);
- Ultraviolett-(UV)-Bestrahlung, Redox- und/oder Wärmebehandlung des funktionalisierten Vorpolymers (Makromer).

**18.** Verwendung eines Polymernetzwerks gemäß Anspruch 7 oder Anspruch 8 als Medikament.

**19.** Vewendung eines funktionalisierten Vorpolymers (Makromer) gemäß einem der Ansprüche 1 bis 6 als Medikament.

## Revendications

**1.** Prépolymère fonctionnalisé (macromère) pouvant être obtenu par réaction d'un prépolymère comprenant au moins un groupe alcool, amine et/ou sulfhydryle, avec un acide dicarbonique monoestérifié insaturé.

**2.** Prépolymère fonctionnalisé (macromère) selon la revendication 1, dans lequel le prépolymère est coiffé en bout avec l'acide dicarbonique monoestérifié insaturé.

**3.** Prépolymère fonctionnalisé (macromère) selon la revendication 1 ou la revendication 2, dans lequel l'acide dicarbonique monoestérifié insaturé est l'acide fumarique monoestérifié.

**4.** Prépolymère fonctionnalisé (macromère) selon l'une quelconque des revendications 1 à 3, dans lequel l'acide dicarbonique monoestérifié insaturé est estérifié avec un alcool alkylique en $C_1$-$C_5$, de préférence un alcool éthylique.

**5.** Prépolymère fonctionnalisé (macromère) selon l'une quelconque des revendications 1 à 4, dans lequel l'acide dicarbonique monoestérifié insaturé est l'ester monoéthylique d'acide fumarique.

**6.** Prépolymère fonctionnalisé (macromère) selon l'une quelconque des revendications 1 à 5, dans lequel le prépolymère est choisi dans le groupe constitué par le polyéthylèneglycol (PEG), le poly(carbonate de triméthylène) (polyTMC), le poly(D,L-lactide) (PDLLA), le poly(L-lactide) (PLLA), le poly(D-lactide) (PDLA), la poly(ε-caprolactone) (PCL), la polydioxanone, et leurs combinaisons.

7. Réseau polymère pouvant être obtenu par polymérisation radicalaire d'un prépolymère fonctionnalisé (macromère) selon l'une quelconque des revendications 1 à 6.

8. Réseau polymère selon la revendication 7, dans lequel la polymérisation radicalaire est une polymérisation radicalaire aux ultraviolets (UV), une polymérisation radicalaire par procédé redox, et/ou une polymérisation radicalaire à la chaleur.

9. Procédé pour former un prépolymère fonctionnalité (macromère), comprenant la réaction d'un prépolymère comprenant au moins un groupe alcool, amine et/ou sulfhydryle avec un acide dicarbonique monoestérifié insaturé.

10. Procédé selon la revendication 9, dans lequel le groupe alcool, amine et/ou sulfhydryle est présent à l'extrémité du prépolymère.

11. Procédé selon la revendication 9 ou la revendication 10, dans lequel l'acide dicarbonique monoestérifié insaturé est l'acide fumarique monoestérifié.

12. Procédé selon l'une quelconque des revendications 9 à 11, dans lequel l'acide dicarbonique monoestérifié insaturé est estérifié avec un alcool alkylique en $C_1$-$C_5$, de préférence un alcool éthylique.

13. Procédé selon l'une quelconque des revendications 9 à 12, dans lequel l'acide dicarbonique monoestérifié insaturé est l'ester monoéthylique d'acide fumarique.

14. Procédé selon l'une quelconque des revendications 9 à 13, dans lequel le prépolymère est choisi dans le groupe constitué par le polyéthylèneglycol (PEG), le poly(carbonate de triméthylène) (polyTMC), le poly(D,L-lactide) (PDLLA), le poly(L-lactide) (PLLA), le poly(D-lactide) (PDLA), la poly(ε-caprolactone) (PCL), la polydioxanone, et leurs combinaisons.

15. Procédé pour former un réseau polymère, comprenant la polymérisation radicalaire d'un prépolymère fonctionnalisé (macromère) tel que défini dans l'une quelconque des revendications 1 à 6.

16. Procédé selon la revendication 15, dans lequel la polymérisation radicalaire est une polymérisation radicalaire aux ultraviolets (UV), une polymérisation radicalaire par procédé redox, et/ou une polymérisation radicalaire à la chaleur.

17. Procédé selon la revendication 15 ou la revendication 16, comprenant :

- la dissolution du prépolymère fonctionnalisé (macromère) dans un solvant convenable ou la mise à disposition d'une masse fondue du prépolymère fonctionnalité (macromère) ;
- le traitement par un rayonnement ultraviolet (UV), procédé redox et/ou à la chaleur du prépolymère fonctionnalisé (macromère).

18. Utilisation d'un réseau polymère tel que défini dans la revendication 7 ou la revendication 8, en tant que médicament.

19. Utilisation d'un prépolymère fonctionnalisé (macromère) tel que défini dans l'une quelconque des revendications 1 à 6, en tant que médicament.

Fig. 1

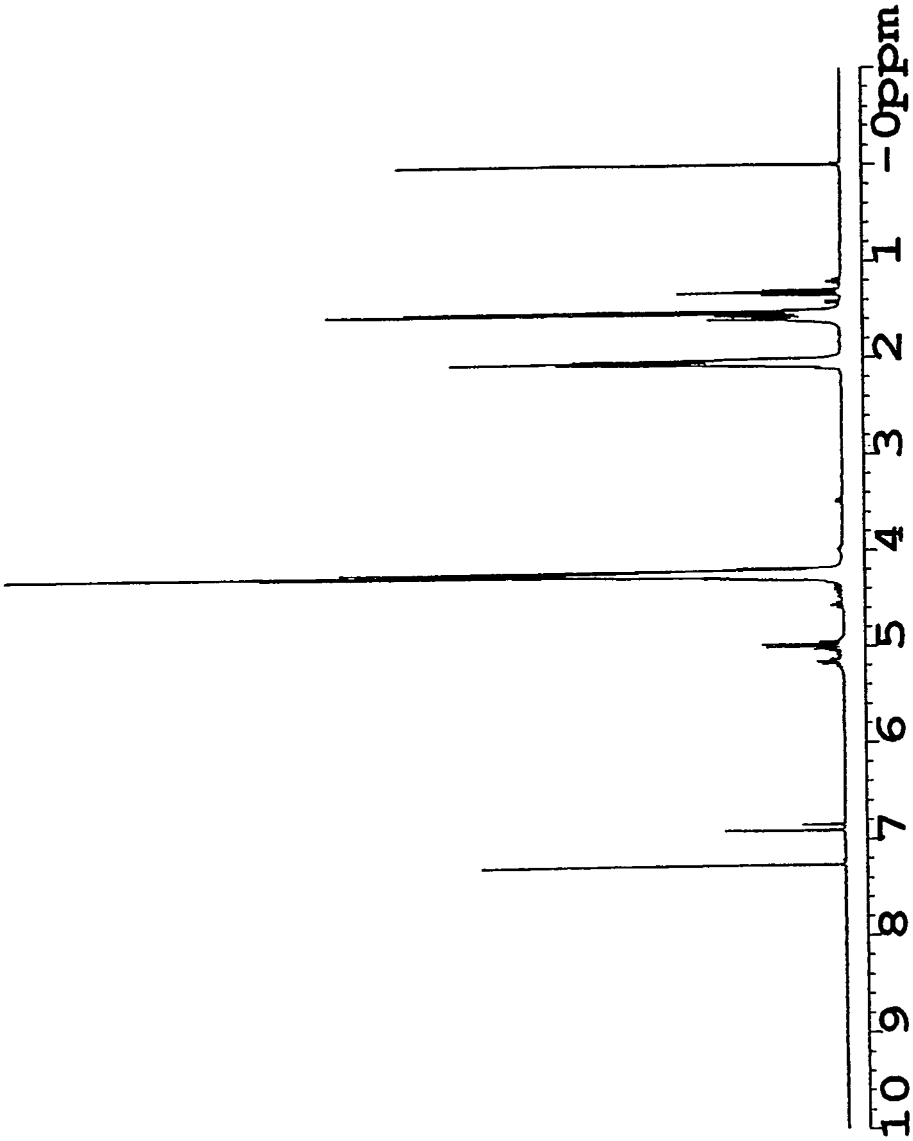

Fig. 2

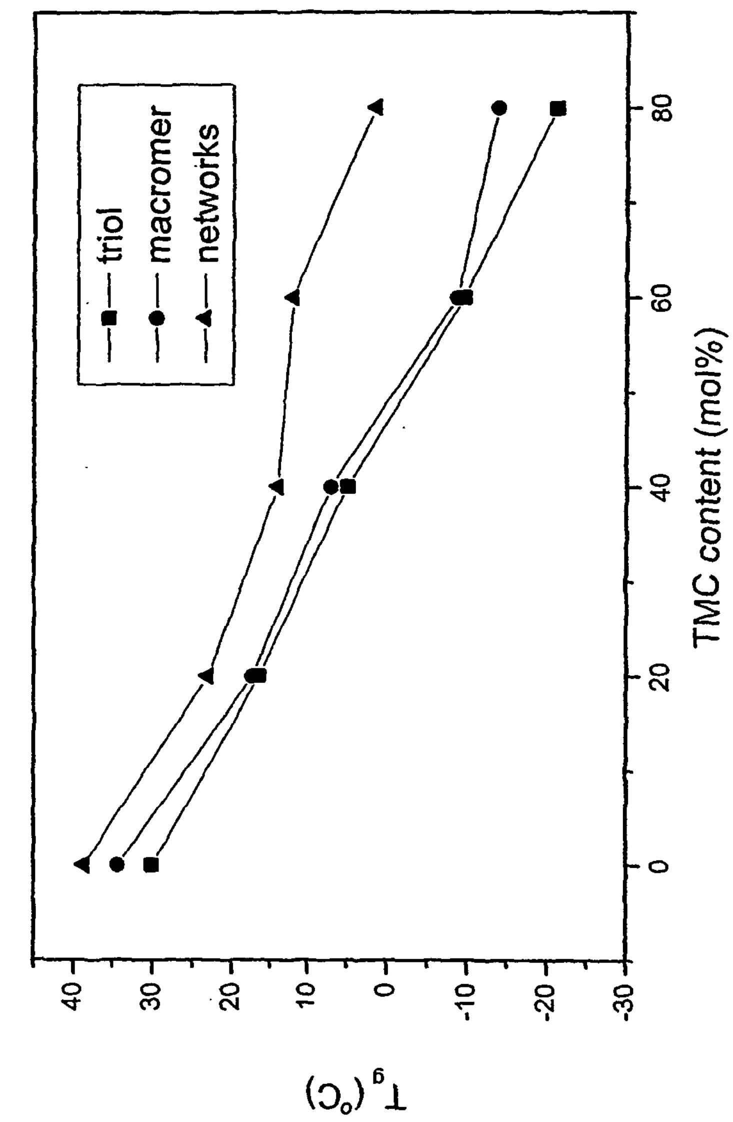
triol
macromer
networks
$T_g$ (°C)
40
30
20
10
0
-10
-20
-30
0
20
40
60
80
TMC content (mol%)

Fig 3

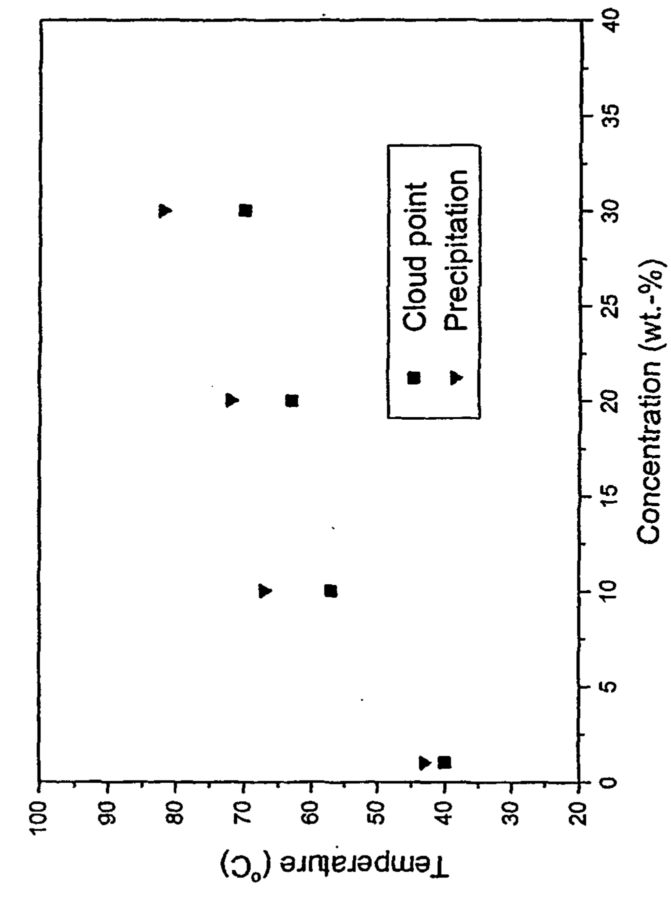
Temperature (°C)
Concentration (wt.-%)
Cloud point
Precipitation
100
90
80
70
60
50
40
30
20
0
5
10
15
20
25
30
35
40

Fig. 4

A

B

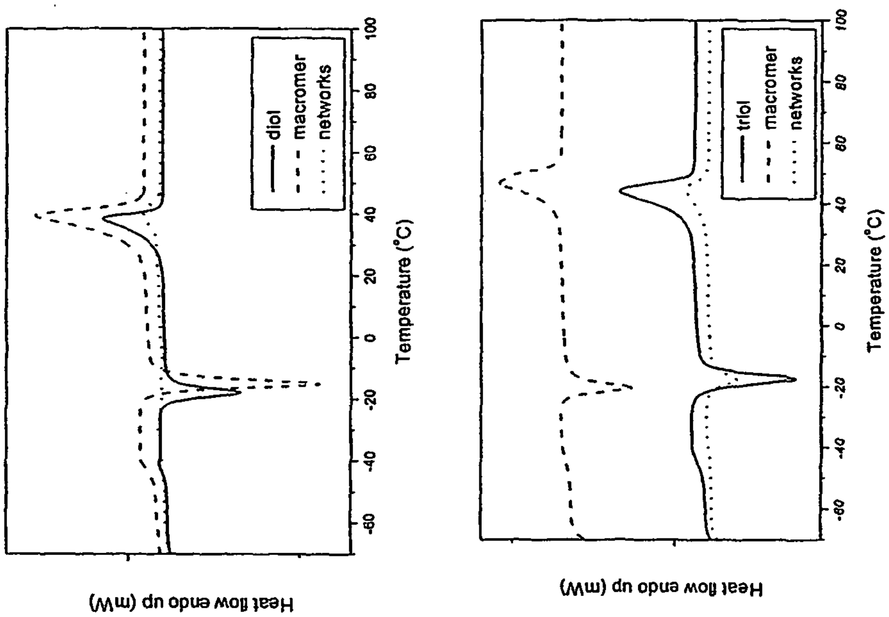
diol
macromer
networks
Heat flow endo up (mW)
Temperature (°C)
triol
macromer
networks
Heat flow endo up (mW)
Temperature (°C)

Fig. 5

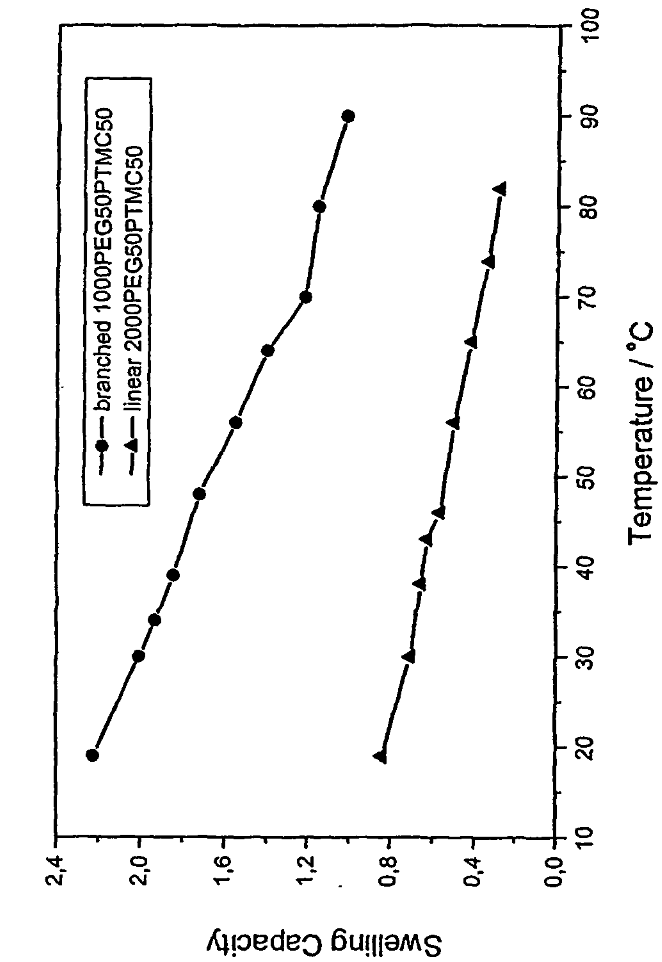
branched 1000PEG50PTMC50
linear 2000PEG50PTMC50
Swelling Capacity
Temperature / °C
0,0
0,4
0,8
1,2
1,6
2,0
2,4
10
20
30
40
50
60
70
80
90
100

Fig. 6

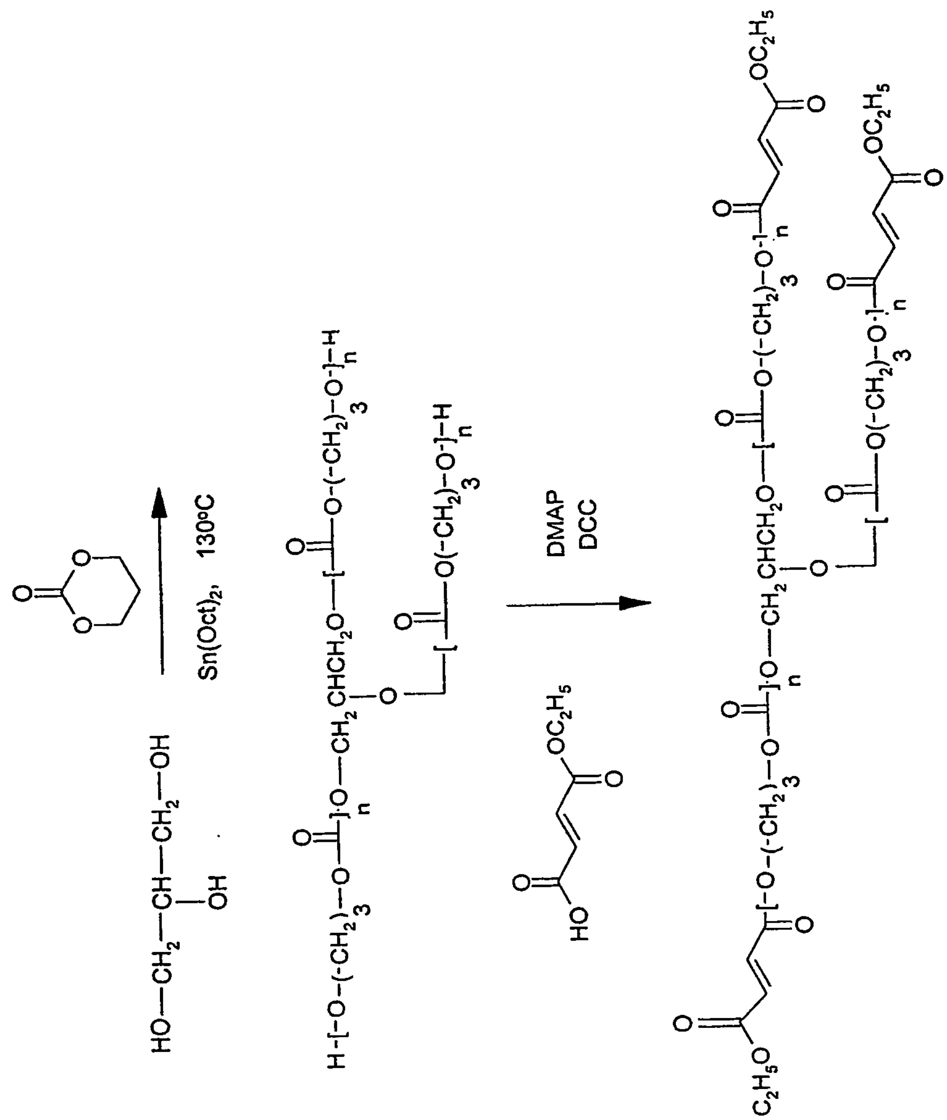

Fig. 7

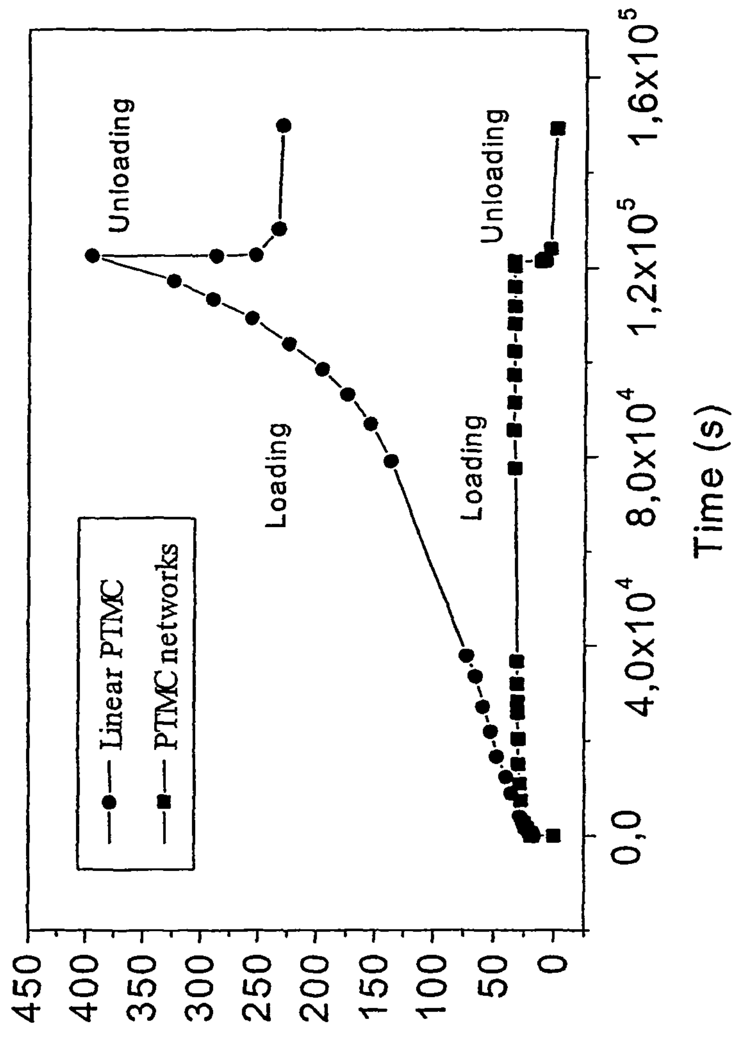
Strain (%)
450
400
350
300
250
200
150
100
50
0
0,0
4,0x10^4
8,0x10^4
1,2x10^5
1,6x10^5
Time (s)
Linear PTMC
PTMC networks
Loading
Loading
Unloading
Unloading

Fig. 8

B

A